# EUROPEAN PATENT APPLICATION

(11) **EP 1 995 540 A2**
(43) Date of publication of application: **26.11.2008**
(21) Application number: 07117563.2
(22) Date of filing: 28.09.2007
(51) Int. Cl.: F26B 9/06, F26B 21/06, F26B 25/00

(54) **Garbage dryer**

(30) Priority: 22.05.2007 KR 20070049747
(71) Applicant: Lee, Hee Ja, Gangnam-gu Seoul 135-870 (KR)
(72) Inventor: Lee, Hee Ja, Gangnam-gu Seoul 135-870 (KR)
(74) Representative: Szynka, Dirk

(57) **Abstract**

The present invention provides a garbage dryer. The garbage dryer detects changes in the temperature inside a dryer using a dielectric sensor by detecting changes in the dielectric constant of garbage and controls a heater and an air circulation fan on the basis of the result of the temperature change detection. Accordingly, the garbage dryer always maintains the inner temperature at a constant temperature and air flow at a constant rate, so that it is possible to increase the drying efficiency and deodorizing efficiency which is attained using an improved deodorizing agent.

## Description

### BACKGROUND OF THE INVENTION

### Field of the Invention

The present invention relates, in general, to a garbage dryer, and more particularly to a garbage dryer, which is capable of drying garbage at increased efficiency by maintaining constant inner temperature and air flow in the garbage dryer using a dielectric sensor, and deodorizing at increase efficiency by using a deodorizing agent having high deodorizing performance and by maintaining the vapor, generated when moisture in garbage evaporates during a process of drying garbage, at a constant volume.

### Description of the Related Art

Garbage (food waste) from households is generally discarded in landfills. This method causes severe environmental harm and is accompanied by severe odors during decomposition. Accordingly, it acts as a substantial factor causing pollution of our surroundings and living environments.

A garbage disposer is a device for treating garbage (food waste) causing such environmental problems. Most conventional garbage disposers adopt a fermentation system using microorganisms. They have an advantage in that they are convenient to use to treat garbage but also have disadvantages in that it is difficult to keep and manage microorganisms and in that the treatment speed is low.

As an alternative, a garbage dryer is a device incorporating a heater provided as a hot wire or a heat-generating element to thereby dehydrate garbage. The dried garbage (food waste) is treated to make fertilizer, or is disposed of in a landfill.

The garbage dryer further incorporates an air fan for sending air to garbage and circulating it therethrough, so that the garbage can be dried effectively and with remarkable efficiency.

There are related arts to this technology, for example, Japanese Patent Publication No. 10-1997-159358 and Korean Patent No. 10-175711. In these documents, an air fan is disposed in the upper portion of a casing provided in a garbage dryer, and an air circulation path is formed in the garbage dryer in order to circulate air from the air fan through the garbage.

Accordingly, the circulating air flow generated by the air fan and the air circulation path repeatedly dries the surface of the garbage over a large area, resulting in increased drying efficiency.

The garbage dryer using a heater has the advantage of treating garbage at high speed but also has a disadvantage in which overheating occurs during a drying process. This means that this device has a safety problem.

On the other hand, the aforementioned other related art garbage dryers dry garbage using only air from an air fan. Accordingly, these are relatively safe in comparison with devices using heaters, but the device using only air takes a long period to perform a drying process, and it is reported that the device does not have as high efficiency as expected.

Furthermore, general garbage dryers exhaust odors through a drain, and thus the dryers are limited to being installed near a sink. The odors in the drain come back from time to time, and thus the odor is inflicted on people.

Korean Patent No. 10-706919 discloses a garbage disposer or a garbage dryer which absorbs and oxidizes odors generated during a garbage decomposition process or a garbage drying process using a deodorizing unit in which a precious metal catalyst is carried in Zeolite or an alumina carrier.

This device has disadvantages in that it is expensive, because a precious metal catalyst is used as a deodorizing agent, which absorbs odors, thus performing deodorizing, and in that it consumes a lot of power because high temperatures are required to maintain high efficiency of the deodorizing agent.

### SUMMARY OF THE INVENTION

Accordingly, the present invention has been made in an effort to solve the aforementioned problems, increase the drying efficiency of garbage by maintaining constant inner temperature and air flow in a garbage dryer, and increase the deodorizing efficiency of a deodorizing agent.

Further, the present invention has been made in an effort to provide a garbage dryer having the advantage of increasing the drying efficiency of garbage by maintaining a constant temperature and air flow using a dielectric sensor, remarkably enhancing safety by suppressing overheating, increasing deodorizing efficiency and prolonging the lifetime of the deodorizing agent by maintaining the vapor, which is generated during a drying process of garbage, at a constant volume.

Still further, the present invention has been made in an effort to provide a garbage dryer having the advantage of increasing the drying efficiency and deodorizing efficiency of a process of drying garbage, so that general households can use the garbage dryers without worrying about the generation of odors.

According to one aspect of the present invention, there is provided a garbage dryer comprising a main body having a drying space inside the main body and having at least one exposed portion of the main body, a cover openably installed on the main body to cover the exposed portion, a drying basket disposed in the main body for receiving garbage therein, a heater installed in the main body for generating heat by being powered by an external power source, an air circulation fan for heating and drying the garbage by transferring a dry air generated by the heater toward the drying basket, a circulation pipe installed in the main body in a manner of communicating with the atmosphere in order to outwardly discharge an air containing a vapor evaporated from the garbage and circulate the dry air toward the drying basket at the same time as a time of performing a process of drying the garbage, a dielectric sensor installed in the main body for controlling the heater and the air circulation fan on the basis of the result of detecting a change in temperature inside the drying space by means of measuring a change in dielectric constant of the garbage, and a deodorizing unit installed outside the main body and incorporating an activated carbon having an average grain size of 0.5 to 1.5 millimeters and supporting copper, manganese, sodium hydroxide and potassium therein in order to absorb and deodorize a vapor containing odorous particles.

Accordingly, garbage from households or restaurants may be received in the garbage basket and dried by the heater and hot air circulating along the circulation pipe by the operation of the air circulation fan. During this drying process, wet air containing an evaporated vapor from the garbage is outwardly discharged through the circulation pipe.

During the drying process, the dielectric sensor can prevent overheating from occurring by detecting a change in dielectric constant, stopping the heater and driving the air circulation fan alone to thereby maintain the temperature constant in the drying space when the volume of vapor is zero, while the dielectric sensor increases the deodorizing efficiency by adjusting the volume of vapor generated by evaporation.

That is, air, in which the volume of vapor is controlled by the dielectric sensor, is absorbed and deodorized by the deodorizing unit, so that odor factors are eliminated. As a result, purified air alone can be emitted to the atmosphere. Accordingly, households and restaurants can use this garbage dryer without worrying about or enduring odors.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 is a perspective view illustrating a garbage dryer according to one embodiment of the present invention;
FIG. 2 is a view illustrating the garbage dryer, the inner structure of which is partially cut away, according to one embodiment of the present invention;
FIG. 3 is an exploded perspective view illustrating a garbage dryer according to one embodiment of the present invention;
FIG. 4 is an exploded perspective view illustrating a drying basket incorporated in the garbage dryer according to one embodiment of the present invention; and
FIG. 5 is a view illustrating a drying process performed in the garbage dryer according to one embodiment of the present invention.

### DETAILED DESCRIPTION OF THE INVENTION

Reference will now be made in greater detail to the garbage dryer according to preferred embodiments of the invention, examples of which are illustrated in the accompanying drawings.

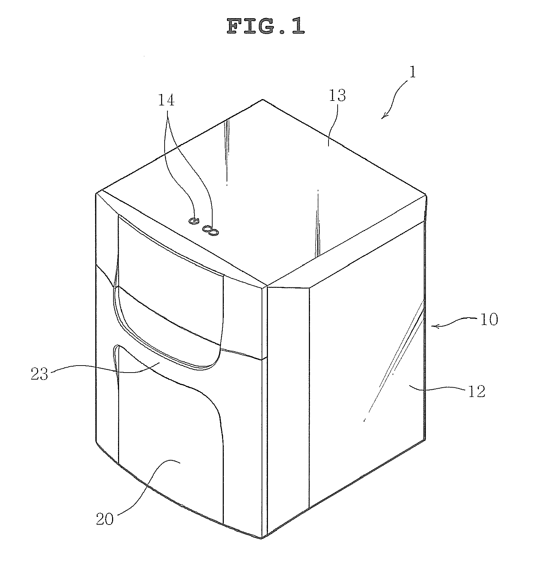

FIG. 1 is a perspective view of the garbage dryer according to one embodiment of the present invention. FIG. 1 illustrates the overall appearance of the garbage dryer, comprising a main body, in which garbage from households and restaurants can be received and dried, and a cover coupled to the front portion of the maul body.

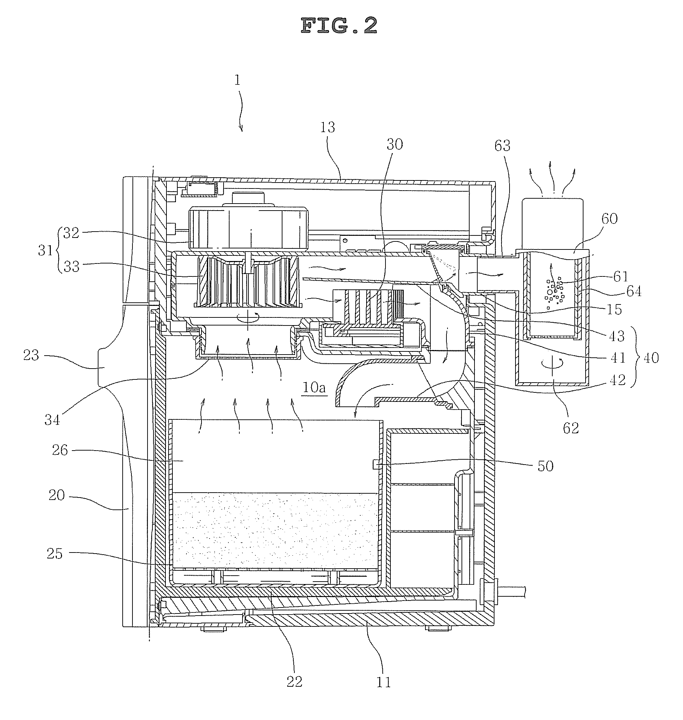

FIG. 2 is a sectional view illustrating the inner structure of the garbage dryer, a portion of which is cut, according to one embodiment of the present invention. FIG. 2 illustrates the structure in which garbage is dried by a heater and hot air circulating through a circulation pipe by the operation of an air circulation fan, and air containing vapor evaporated during the garbage drying process is exhausted through the air circulation pipe.

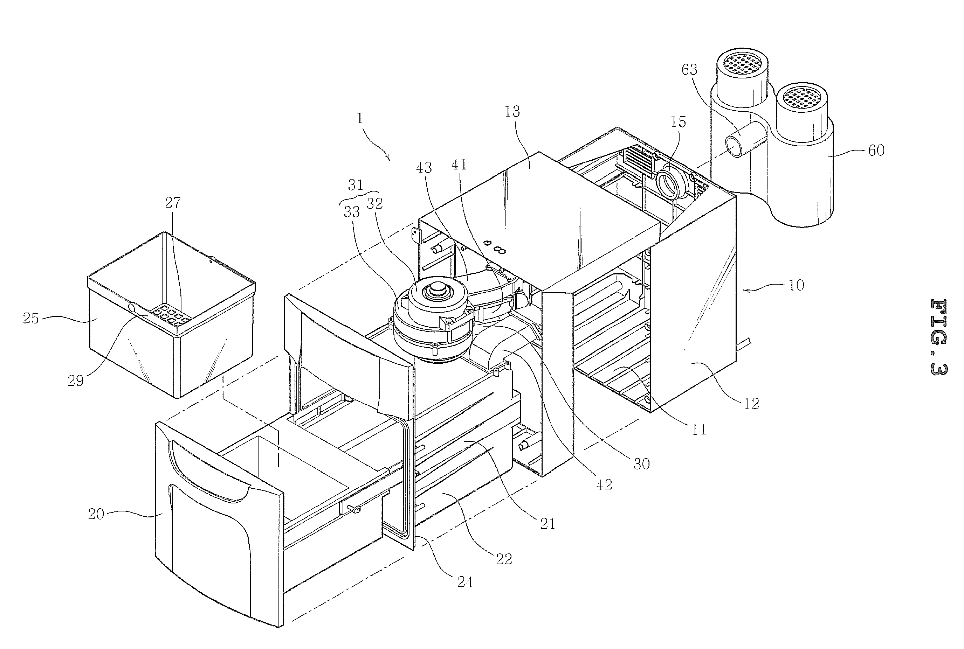

FIG. 3 is an exploded perspective view illustrating the overall appearance of the garbage dryer according to one embodiment of the present invention. FIG. 3 illustrates the positional and coupling relationship of components, the heater for drying the garbage using hot air, the air circulation fan, and the air circulation pipe for outwardly discharging an evaporated vapor generated during the garbage drying process, to the atmosphere.

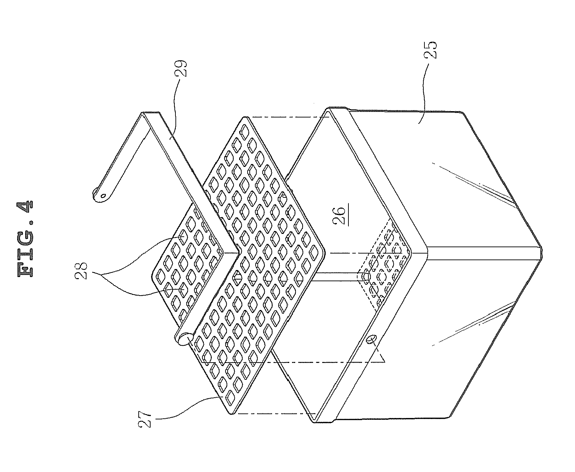

FIG. 4 is an exploded perspective view illustrating the drying basket incorporated in the garbage dryer according to one embodiment of the present invention. Provided are a support frame for increasing the efficiency of air passage of the drying basket, having garbage therein, and a separation member for separating the drying basket from the garbage dryer after the completion of the garbage drying process.

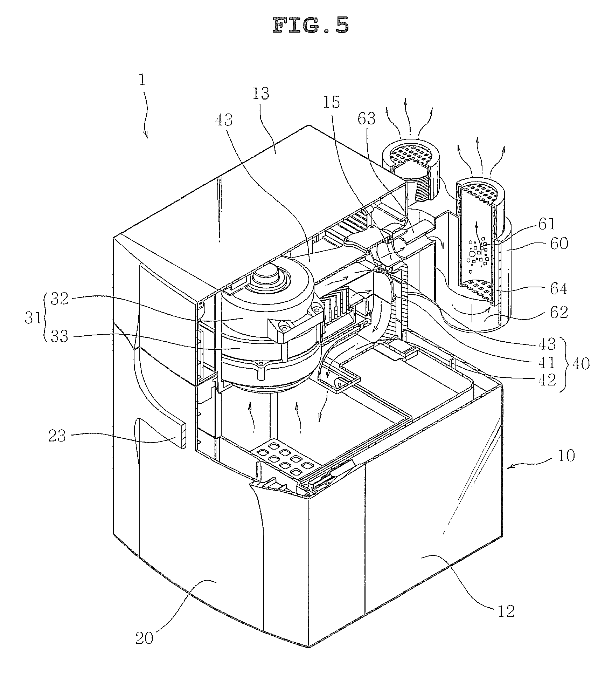

FIG. 5 is a sectional view illustrating the garbage drying process performed in the garbage dryer, part of which is cut away, according to one embodiment of the present invention. With reference to FIG. 5, the garbage in the garbage basket is dried by hot air, and an evaporated vapor from the garbage is outwardly discharged to the atmosphere.

As shown in FIG. 1 and FIG. 2, the present invention relates to a garbage dryer 1, which is capable of increasing the drying efficiency of garbage using the dielectric sensor 50, which can detect changes in both temperature and dielectric constant, and which is capable of increasing the deodorizing efficiency of an evaporated vapor generating during the garbage drying process, and containing odorous particles.

A main body 10 is a casing that defines the overall appearance of the garbage dryer 1. A drying space 10a is provided in the main body 10. The main body 10 has at least one exposed portion.

In this embodiment, the main body 10 comprises a bottom plate 11, side plates 12 provides at edges of the bottom 11, and an upper plate 13. The drying space 10a is defined by the bottom plate 11, the side plates 12 and the upper plate 13. hi this embodiment, a front face is open. However, this structure is an example, and the upper face or the side face can be open, instead of the front face.

A manipulation switch 14 for driving the garbage dryer 1 is provided on the upper plate 13 of the main body 10. As the manipulation switch 14, a power switch and an operation switch, which displays the operation state, are attached on the upper plate 13 of the main body 10.

A cover 20 is provided in a manner such that it usually closes the exposed portion of the main body 10, so that an air containing odorous particles from garbage in the main body 10 is outwardly exhausted to the atmosphere. According to necessity, the cover 20 is opened and garbage is introduced into the drying space 10a of the main body 10, so that the garbage is received in a garbage basket 25 which will be described below.

The cover 20, according to a preferred embodiment, as shown in FIG. 3, is installed in a manner such that the cover 20 can slide along a guardrail 21 provided on the front face, so that the cover 20 can be smoothly opened.

The cover 20 according to a more preferable embodiment has a support 22 thereon, so that the drying basket 25 can be placed on the support 22. By this configuration, the drying basket 25 can be naturally pulled out when the cover 20 is opened.

The cover 20 according to still further preferable embodiment, has a knob or a handle 23 on the other surface thereof, so that the opening and closing of the cover 20 can be easily performed using the handle 23.

A sealing member 24 is provided between the cover 20 and the main body 10 in order to completely seal the drying space 10a when the cover 20 is closed and prevent noise from being generated when the cover 20 is brought into contact with the main body 10.

Accordingly, the cover 20 is usually in a state such that it closes the exposed portion of the main body 10, and is opened when garbage is put in, so that it is possible to prevent an air containing odorous particles from escaping from the drying space 10a during the garbage drying process, and it is also possible to increase drying efficiency because the drying space 10a is maintained in a sealed state.

The drying basket 25 has a receiving space 26 and is placed on the support 22 formed on the cover 20. The drying basket 25 plays a role of storing garbage in the receiving space 26 thereof

According to the embodiment, an additional support frame 27 is installed on the bottom of the inner space of the drying basket 25, so that the drying efficiency of garbage received in the receiving space 26 can be increased.

That is, the shape of the support frame 27, as shown in FIG. 4, is the same or almost the same as that of the receiving space 26, and has a plurality of ventilation holes 28. When the support frame 27 is installed on the bottom of the inner space of the drying basket 25, air can pass through the garbage received in the garbage basket, and thus the drying efficiency is increased.

According to this embodiment, a separation member 29 is rotatably installed in the drying basket 25. Accordingly, it is possible to easily separate the drying basket 25 from the main body 10 when discharging the dried garbage by holding the separation member 29.

In the bottom portion of the drying basket 25, an agitator (not shown) can be provided for eliminating the difference in temperatures at an upper portion and a lower portion of the garbage and smoothly eliminating moisture by agitating the garbage.

The heater 30 generates heat by being powered by an external power source, and dries the garbage received in the garbage basket 25. For this, the heater 30 is positioned in the upper portion of the drying basket 25 and is installed in the drying space 10a of the main body 10.

Any known configuration can be applied to the heater 30 as long as it comprises a heating element that can generate heat when powered by an external power source. According to a preferred embodiment of the present invention, a positive temperature coefficient heater (PTC heater) can be provided as the heater 30, so that the garbage is maintained within a set temperature range.

The temperature range may be from 60 to 70 °C.

Here, the external power source for driving the heater 30 may be a battery pack. Further, the heater 30 can be powered from an external power source in a manner such that a plug connected to the back surface of the main body 10 by an electric wire is received in a receptacle when the garbage dryer is used in a household or a restaurant.

That is, when the garbage dryer is used at a place equipped with receptacles, such as households or restaurants, the heater is driven by plugging a plug into a receptacle. Alternatively, when the garbage dryer is used at a place where there are no receptacles, the garbage dryer 1 is driven by a battery pack.

This external power source plays a role of supplying electric power to the manipulation switch 14, to an air circulation fan 31, which will be described below, and to a variety of electric components in addition to the heater 30.

The air circulation fan 31 plays a role of blowing heat generated from the heater 30 toward the drying basket 25 to thereby dry the garbage.

For this, the air circulation fan 31 comprises a driving motor 32 and a driving fan 33, coupled to the driving motor 32 by a shaft, and is installed at a place near the heater 30. According to a preferred embodiment, the air circulation fan 31 is installed above and in front of the heater 30.

Accordingly, air is blown toward the drying basket 25 and is passed through the heater 30 by the air circulation fan 31. When the air is blown to the drying basket 25, the air is heated by the heater 30, and the garbage in the garbage basket 25 is dried by the hot air.

Under the driving fan 33, constituting the air circulation fan 31 a strainer 34 is installed in order to prevent garbage from invading into the driving fan 33, attributable to the fact that the garbage is boiled by the hot air during the drying process.

The circulation pipe 40 outwardly discharges an air containing an evaporated vapor generated while the garbage is dried by hot air blown by the air circulation fan 31, to the atmosphere, and circulates dry air through the drying basket 25 using the structure in which it can communicate with atmosphere and is installed in the drying space 10a of the main body 10.

According to this embodiment, the circulation pipe 40 comprises a discharge pipe 41, a blowing pipe 42 and an evacuating pipe 43. The discharge pipe 41 is installed near the heater 30 so as to receive hot air containing heat which is generated by the heater 30 and transfer the hot air. The blowing pipe 42, connected to the discharge pipe 41, is installed near the drying basket 25 so as to blow the hot air transferred from the discharge pipe 41 to the drying basket 25.

The evacuating pipe 43 has a first end disposed near the air circulation fan 31 and a second end fixed to the back of the main body 10 in order to finally evacuate the air containing vapor evaporated from the garbage.

In the case in which an additional evacuating portion 15 is provided at the back of the main body 10, the evacuating pipe 43 is connected to the evacuating portion 15 to outwardly discharge the air containing the vapor.

The circulation pipe 40, constituted by the discharge pipe 41, the blowing pipe 42 and the evacuating pipe 43, is installed throughout the entire drying space 10a of the main body 10. Accordingly, it is preferable that the pipes be provided in a separated form, but the invention is not necessarily limited to this configuration.

Since the circulation pipe 40, constituted by the discharge pipe 41, the blowing pipe 42 and the evacuating pipe 43, is installed in the drying space 10a of the main body 10, hot air, obtaining heat from the heater 30, is continuously supplied to the drying basket 25, so that the garbage is dried.

During this procedure, wet air containing vapor is evacuated outside the garbage dryer, and dry air is circulated to the garbage dryer, to thereby increase drying efficiency and heat efficiency.

The dielectric sensor 50 is installed in the main body to detect changes in temperature in the drying space 10a by detecting changes in dielectric constant of the garbage. On the basis of the result of the detection, the dielectric sensor 50 controls the heater 30 and the air circulation fan 31.

According to the embodiment, the dielectric sensor 50 is installed in the drying basket 25 containing the garbage therein to detect changes in the temperature inside the drying space 10a by detecting changes in the dielectric constant of the garbage.

The dielectric sensor 50 is a sensor that can detect changes in dielectric constant using electromagnetic waves and can estimate the moisture content in vapor by calculating changes in dielectric relaxation frequencies.

As the moisture content of vapor increases, the dielectric relaxation frequency increases. Accordingly, at a high dielectric relaxation frequency, the dielectric sensor 40 keeps driving the heater 30 because it is determined that the garbage contains a large amount of moisture. On the other hand, when there is no moisture in vapor, the dielectric relaxation frequency decreases, so that the heater 30 is stopped by the dielectric sensor 50.

The most important advantage of the dielectric sensor 50 is that it is capable of simultaneously checking temperature and dielectric constant. According to the principle of the dielectric sensor 50, a high dielectric constant means that electric energy is basically transferred well. That is, in soil, which has a low dielectric constant, electric current does not flow very well, and electromagnetic waves penetrate the soil. However, when soil is wet and the dielectric constant of the soil is increased, electric current starts to flow, and electromagnetic waves cannot penetrate the wet soil very well.

By using the dielectric sensor 50, which uses mis principle, precision is increased at low cost.

Accordingly, since changes in the temperature of the drying space 10a are detected by detecting changes in the dielectric constant of garbage using the dielectric sensor 50, and the heater 30 and the air circulation fan 31 are controlled on the basis of the detection of changes in temperature, it is possible to increase the deodorizing efficiency of a deodorizing unit, which will be described below, as well as drying efficiency.

The deodorizing unit 60 is connected to the exhausting portion 15 installed to the back of the main body 10, and incorporates a deodorizing agent 61 therein. Accordingly, the deodorizing unit 60 absorbs vapor containing odorous particles, which is outwardly discharged through the exhausting portion 15.

Odors from the garbage dryer 1 are caused by the garbage. Accordingly, if the moisture content in the garbage is high, the odors include both acidic and basic odorous particles.

Accordingly, the deodorizing agent 61 built into the deodorizing unit 60 must have high instant absorption performance and high selective absorption performance at high moisture content, and it is necessary to absorb, oxidize and neutralize both basic and acidic odorous particles.

The deodorizing agent 61 is provided as activated carbon having an average grain size of 0.5 to 1.5 millimeters in consideration of the smooth flow of discharge gas and the absorption performance of the deodorizing agent 61 for the following reasons.

When the grain size is less than 0.5 millimeters, the flow of the discharge gas is suppressed, resulting in an increase in the temperature inside the garbage dryer 1. Conversely, when the grain size is larger than 1.5 millimeters, the flow of the discharge gas becomes faster, and consequently there is the possibility that absorption, oxidation and neutralization will not be performed very well.

In addition, in order to eliminate acidic odorous particles and increase deodorizing efficiency, copper Cu, manganese Mn, sodium hydroxide NaOH and potassium hydroxide KOH are impregnated in activated carbon.

That is, when the activated carbon, having the aforementioned grain size is applied to the garbage dryer 1 according to the present invention, odors are not generated due to absorption at an early state. However, after a predetermined period (three days or more) has elapsed, acidic odors are generated.

Accordingly, in order to eliminate acidic odors and increase absorption performance, it is preferable that metals, such as copper Cu and manganese Mn, and neutralizing agents, such as sodium hydroxide NaOH and potassium hydroxide KOH, be impregnated in the activated carbon.

According to the present invention, if manganese Mn, having strong oxidizing power, is added in the form of an oxide, the usage is preferably in the range of 5000 to 80000 mg/l. If the usage is less than 5000mg/l, the oxidizing power is low. On the contrary, if the usage is more than 80000mg/l, manganese is provided in excess of saturation amount, and thus it cannot be completely dissolved.

Copper Cu is also added in the form of an oxide and has excellent oxidizing power and an excellent ammonia deodorizing function. The usage of copper is preferably in the range of 5000 to 200000mg/l. If the usage is less than 5000mg/l, deodorizing performance is low. On the contrary, if the usage is larger than 200000mg/l, deodorizing efficiency is not increased in proportion to the usage.

Sodium hydroxide NaOH and potassium hydroxide KOH are added for the purpose of neutralization, and the combined usage thereof is preferably in the range of 100 to 10000mg/l. If the usage is less than 100mg/l, neutralizing power is low. On the contrary, if the usage is larger than 10000mg/l, neutralizing efficiency is not increased in proportion to the usage.

The deodorizing agent 61 according to the embodiment has high oxidizing power and high neutralizing performance, because manganese and copper, having high oxidizing power, and sodium hydroxide and potassium hydroxide, having high neutralizing performance, are impregnated into activated carbon. Accordingly, it is possible to enhance the efficiency of elimination of acidic odors.

The usage of the activated carbon is in proportion to the volume of the garbage to be treated. For example, in order to dry 100g of garbage, the usage of the activated carbon is preferably 100 to 500 grams. The usage can vary according to the type of garbage (food waste).

In order to prepare the aforementioned deodorizing agent, copper, manganese, sodium hydroxide and potassium hydroxide are dissolved in 1 liter of water, activated carbon is added to the water in order to impregnate copper, manganese, sodium hydroxide and potassium hydroxide in activated carbon, and then activated carbon is dehydrated. After that, the activated carbon is rapidly dried, without causing deformation to the activated carbon, at 90 to 120°C. This impregnation method is well known to people having ordinary skill in the art.

The reason that the components copper, manganese, sodium hydroxide and potassium hydroxide are impregnated in activated carbon at room temperature is to avoid deformation of the pore structure of activated carbon and a decrease in the surface area thereof. When activated carbon is processed at high temperature, the pore structure of activated carbon can be deformed, and particles are formed on the surface of the activated carbon while the activated carbon is cooled to room temperature, resulting in a decrease in the surface area of activated carbon.

As described above, odors generated from general garbage dryers which are difficult to treat with general activated carbon can be treated by the garbage dryer according to the present invention by controlling the emission of vapor and odors by using the dielectric sensor 50 and optimizing absorption, oxidation and neutralization performance using the activated carbon in which a catalyst is impregnated.

In the deodorizing unit 50 incorporating such activated carbon according to a preferred embodiment of the invention, as shown in FIG. 5, a deodorizing space 62 is separately provided in the deodorizing unit 60, and is filled with the activated carbon, so that wet air containing vapor introduced through a suction hole 63 is absorbed and deodorized on both sides of the deodorizing space 62.

The reason that the deodorizing space 62 is separately provided in the deodorizing unit 60 is to increase the sectional area through which odorous particles pass and to increase the time that the odors are retained, thereby increasing the absorption and deodorizing efficiency of odorous particles by the activated carbon.

According to more preferable embodiment of the present invention, a deodorizing cylinder 64 is separately provided in the deodorizing space 62, so that the activated carbon, which has reduced performance after it has been used for a predetermined period, can be easily and simply replaced with new material by an operator.

Since the deodorizing unit 60 is connected to the evacuating portion 15 installed to the back of the main body 10, vapor containing odorous particles outwardly discharged through the evacuating portion 15 is distributed and then supplied to the deodorizing space 62, which is separately provided in the deodorizing unit 60 through the suction hole 63. During this procedure, odorous particles are eliminated by the activated carbon incorporated in the deodorizing space 62, and clean air is emitted to the atmosphere.

The process of drying garbage using the garbage dryer 1 having the deodorizing unit 60 will be described in detail below.

After garbage is introduced into the garbage basket 25 and the cover 20 is closed, an operator may manipulate the manipulation switch 14. At this time, power is supplied through the electric wire connected to the back of the main body 10 from an external power source, and the air circulation fan 31 and the heater 30, installed in the drying space 10a of the maul body 10, are driven.

Air, generated by driving the air circulation fan 31, is transferred to the discharge pipe 41 while gaining heat from the heater 30 to thus be hot air, is blown to the blowing pipe 42 connected to the discharge pipe 41, and is then blown to the drying basket, so that the garbage in the garbage basket is dried by the hot air.

During this procedure, changes in the dielectric constant of the garbage are continuously detected by the dielectric sensor 50, and thus changes in the temperature inside the drying space 10a are detected. On the basis of the detection of the changes in temperature, the operation of the heater 30 and the air circulation fan 31 are controlled.

While drying the garbage using the heater 30, an air containing an evaporated vapor from the garbage, is supplied to the evacuating pipe 42 due to the air circulation in the air circulation pipe 40, and is then supplied to the deodorizing unit 60, installed downstream of the main body 10, via the evacuating portion 15. As a result, the vapor is absorbed to the deodorizing agent 61 incorporated in the deodorizing unit 60.

Accordingly, the vapor absorbed by the deodorizing unit 60 is decomposed in the activated carbon, is then dried by dry air continuously outwardly discharged from the main body 10, and is finally outwardly discharged to the atmosphere.

The deodorizing agent will be used for at least three months, but normally for six months.

As described above, the garbage dryer according to the present invention can increase the drying efficiency of garbage by detecting changes in the dielectric constant of garbage using the dielectric sensor to detect changes in the temperature inside the garbage dryer and by controlling the operation of the heater and the air circulation fan on the basis of the detection, thereby maintaining the temperature and air flow in the garbage dryer constant.

Since the changes in the temperature inside the garbage dryer are always monitored and the temperature is maintained constant, overheating of the heater is prevented, so that safety in the use of the garbage dryer can be ensured.

Since the volume of an evaporated vapor generated during the drying process is maintained constant by controlling the temperature and air flow, the deodorizing and absorption performances of the deodorizing unit can be enhanced.

Accordingly, since the garbage dryer according to the present invention has high drying efficiency and high deodorizing efficiency with respect to odorous particles, it can be used in households and restaurants without worrying about odor generation, and thus the present invention is very useful.

Although a preferred embodiment of the present invention has been described for illustrative purposes, those skilled in the art will appreciate that various modifications, additions and substitutions are possible, without departing from the scope and spirit of the invention as disclosed in the accompanying claims.

## Claims

1. A garbage dryer, comprising:
a main body having a drying space inside the main body and having at least one exposed portion of the main body;
a cover openably installed on the main body to cover the exposed portion of the main body;
a drying basket installed in the main body for receiving garbage therein;
a heater installed in the main body for generating heat when powered by an external power source;
an air circulation fan for heating and drying the garbage by transferring a dry air generated by the heater toward the drying basket;
a circulation pipe installed in the main body in a manner of communicating with the atmosphere in order to outwardly discharge an air containing a vapor evaporated from the garbage and circulate the dry air toward the drying basket at the same tune as a time of performing a process of drying the garbage;
a dielectric sensor installed in the main body for controlling the heater and the air circulation fan on the basis of the result of detecting a change in temperature inside the drying space by means of measuring a change in dielectric constant of the garbage; and
a deodorizing unit installed outside the main body and incorporating an activated carbon having an average grain size of 0.5 to 1.5 millimeters and supporting copper Cu, manganese Mn, sodium hydroxide NaOH, and potassium hydroxide KOH therein, in order to absorb and deodorize a vapor containing odorous particles.

2. The garbage dryer according to claim 1, wherein support frames for increasing air transmission rate have a plurality of ventilation holes and are installed in a bottom portion of an inner space of the drying basket in a manner of being spaced apart from the bottom portion.

3. The garbage dryer according to claim 1 or 2, wherein the deodorizing unit has deodorizing spaces filled with an activated carbon, which are separately provided in the deodorizing unit, so that the air containing a vapor introduced through a suction hole is distributed toward the deodorizing spaces, and the distributed air is respectively absorbed and deodorized at the deodorizing space.

4. The garbage dryer according to any one of claims 1 to 3, the activated carbon is prepared in a manner such that 5000 to 80000 mg of manganese, 5000 to 200000 mg of copper, 100 to 10000 mg of sodium hydroxide and 100 to 10000 mg of potassium hydroxide are dissolved in 1 liter of water, activated carbon having an average grain size of 0.5 to 1.5 millimeters is added to the water, the activated carbon is dehydrated, and then the activated carbon is dried at a temperature in the range from 90 to 120 °C.
